(19) Europäisches Patentamt
European Patent Office
Office européen des brevets

(11) **EP 2 991 604 B1**

(12) **EUROPEAN PATENT SPECIFICATION**

(45) Date of publication and mention
of the grant of the patent:
**28.11.2018 Bulletin 2018/48**

(51) Int Cl.:
*A61F 13/49* *(2006.01)*

(21) Application number: **14725381.9**

(22) Date of filing: **02.05.2014**

(86) International application number:
**PCT/EP2014/059012**

(87) International publication number:
**WO 2014/177705 (06.11.2014 Gazette 2014/45)**

(54) **ABSORBENT ARTICLE**

SAUGFÄHIGER ARTIKEL

ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.05.2013 EP 13166290**

(43) Date of publication of application:
**09.03.2016 Bulletin 2016/10**

(73) Proprietor: **Ontex BVBA**
**9255 Buggenhout (BE)**

(72) Inventors:
• **HANSSEN, TOM**
**B-9255 Buggenhout (BE)**
• **DE POORTER, Annick**
**B-9255 Buggenhout (BE)**
• **VAN GOETHEM, Jeroen**
**B-9255 Buggenhout (BE)**

(74) Representative: **Macchetta, Andrea et al**
**Ontex BVBA**
**Korte Keppestraat 21**
**9320 Aalst (BE)**

(56) References cited:
**US-A1- 2009 187 157**

## Description

## Field of the invention

The present invention relates to absorbent articles, preferably a disposable absorbent article, such as a diaper.

## Background

Generally known disposable absorbent articles, such as diapers, training pants, incontinence articles, comprise a front belly section, a rear back section and a crotch section extending in the longitudinal direction between said front belly section and said rear back section. The crotch section is permanently attached to the belly section and to the back section. The crotch section as well as the belly section and the back section define the leg openings of the incontinence article. Absorbent articles typically include stretchable materials, such as elastic strands, to provide a snug fit and a good seal of the article. Said stretchable material may be present in the front belly section and/or the rear back section and/or the crotch section and/or the leg openings of said article.

US 2009/187157 provides an incontinence article in the form of pants, said article comprises a front belly section; a rear back section, said rear back section connected to said front belly section at side seam areas on both sides during production to form a belly and back band which is continuous in a transverse or peripheral hip direction to define a hip opening which is closed in said peripheral hip direction; a crotch section having an absorbent body, said crotch section extending in a longitudinal direction between and permanently attached to said belly section and said back section. The crotch section, the belly section and the back section define leg openings of the incontinence article. The belly section and the back section having an edge contour, which extends towards a transverse center axis of said crotch section and which differs from said transverse or peripheral hip direction to define said leg openings. The article comprises first elastification means disposed in said belly section and said back section, said first elastification means extending at a separation from and parallel to each other in said transverse or peripheral hip direction to thereby extensively elasticise said belly section and said back section. The article further comprises second elastification means disposed in an area of said belly section and said back section on a crotch side facing said leg openings, said second elastification means extending from said two side seam areas towards a longitudinal center axis of the incontinence article in a curved shape, thereby fanning out with increasing separation from each other in a first area of said belly section and said back section on said crotch side facing said leg openings. With this configuration, the first restoring force generated through stretching of said first area decreases towards said crotch section. This is disadvantageous as, when worn, the absorbent article will not closely and tightly fit the wearer's body and thereby provide a non-comfort feeling to said wearer. The non-tight absorbent article and thereby the uncomfortable feeling is particularly located at the front section on the crotch side facing the leg openings due to the anatomy of the human's body. Indeed, with respect to the protruding curved anatomy of the human body at the back section on the crotch side facing the leg openings (at the buttocks), elastified means fanning out with increasing separation from each other will fit, decrease the tension on the wearer's skin thereby providing him a comfort feeling. In contradiction, with respect to the anatomy of the human body at the front section on the crotch side facing the leg openings, elastified means fanning out with increasing separation from each other will not closely fit to the wearer's skin thereby providing him a non-comfort feeling.

In addition, elastified means fanning out with increasing separation from each other at the front section on the crotch side facing the leg openings produce a high amount of frills, i.e. a large number of folds or crimps per centimeter. This three-dimensional wavy structure is a bulky indiscrete structure that causes relative motions, in particular, when the user is highly mobile, which irritates the skin surface and cause unpleasant or even medically problematic skin irritations.

The object of the present invention to overcome at least part of the above mentioned problems. The present invention aims at providing an improved absorbent article of the above-described type with regard to wear comfort. This object is achieved in accordance with the invention by a disposable absorbent article comprising the features of claim 1.

## Summary

The present invention provides a disposable absorbent article, suitable to be worn about the lower torso of a wearer, comprising a front belly section and a rear back section, each of said section is provided with longitudinal edges and a transversal edge. The article further comprises a crotch section extending between the front belly section and the rear back section and is permanently attached to the front belly section and the rear back section. The crotch section comprises an absorbent body and extends in the direction of a longitudinal center axis of the absorbent article. The crotch section, the front belly section and the rear back section define leg openings of the absorbent article and the front belly section and the rear back section have an edge contour which extends starting from the longitudinal edges towards a transverse center axis of the crotch section. The front belly section and the rear back section comprise first elastification means, and, in an area of the front belly section and the rear back section on the crotch side facing the leg openings, second elastification means are provided. Said second elastification means extend starting from longitudinal edges towards the longitudinal center axis of the absorbent article in a curved shape and are fanning out with increasing separation from each other in the rear back section and with equal separation from each other and parallel to each other in the front belly section.

Providing the area of the front belly section, on the crotch side facing the leg openings, with second elastification means which are fanning out with equal separation from each other and parallel to each other presents several advantages. For instance, said area will closely fit the wearer's body and will have an automatic uplift close to the crotch area. Furthermore, due to the relatively small body zone that has to be covered by the area of the front belly section, on the crotch side facing the leg openings, the number of folds/crimps will be reduced compared to an absorbent article wherein the elastic means are fanning out with increasing separation at the front section.

In a preferred embodiment, the crotch section comprises leg elastification means which extend outside of the absorbent body along the leg opening and terminate in the longitudinal direction in the front belly section and in the rear back section. In a preferred embodiment, the leg elastification means terminate in the longitudinal direction in the area of the front belly section and the rear back section wherein second elastification means are provided. Said leg elastification means provide reliable side leakage protection.

In a preferred embodiment, the first elastification means extend, at a separation from each other and parallel to each other and parallel to the transversal edge thereby extensively elasticising the front belly section and the rear back section.

In a preferred embodiment, the front belly section and/or the rear back section further comprise an elastified material provided on 1% to 65%, preferably 20% to 60%, more preferably on 40 to 55%, most preferably on at least 50% of the surface area of said front belly section and/or the rear back section.

In a preferred embodiment, the fanning-out degree of the second elastification means in the rear back section is larger than the fanning-out degree of the second elastification means in the front belly section. This is advantageous as, in the rear back section, the restoring force of the second elastification means is reduced within the area on the crotch side facing the leg openings. Said restoring force is reduced in the direction from the longitudinal edge towards the crotch section which provides a better fit of the absorbent article to the anatomy of the wearer's body.

In a preferred embodiment, the separation between the second elastification means of the front belly section is 3 to 20 mm, preferably 4 to 15 mm, more preferably 5 to 10 mm, most preferably 6 to 8 mm.

In a preferred embodiment, the crotch section comprises a liquid-impermeable back sheet material and a top sheet material between which the absorbent body is disposed, and the back sheet material and/or the top sheet material form a projection that extends past the absorbent body in the transverse direction and on both sides of said absorbent body. In a further preferred embodiment, the absorbent body is at least partially covered by a high loft material.

In a preferred embodiment, the surface proportion of the crotch section with respect to the entire surface of the absorbent article is of from 25 to 55%.

In a preferred embodiment, the crotch section overlaps at least 12% of the surface of the front belly section and at least 20% of the surface of the rear back section in an extended state of the absorbent article.

In a preferred embodiment, the absorbent body overlaps at least 5% of the surface of the front belly section and/or at least 10% of the surface of the rear back section.

## Brief description of the figures

Fig. 1 shows a top view of a first embodiment of a disposable absorbent article according to the present invention.

Fig. 2 shows a side view in the longitudinal direction (L) of the disposable absorbent article of Fig. 1.

Fig. 3 shows a side view in the width direction (W) of the disposable absorbent article of Fig. 1.

Fig. 4 shows a top view of a second embodiment of a disposable absorbent article according to the present invention.

Fig. 5 shows a side view in the longitudinal direction (L) of the disposable absorbent article of Fig. 4.

Fig. 6 shows a side view in the width direction (W) of the disposable absorbent article of Fig. 4.

## Detailed description

The present invention relates to an absorbent article having at least improved elastic properties and wear comfort. The absorbent article of the present invention can be a diaper used for babies, children or any adult person who is in need of such article, for instance a sick person or an old person.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight" (weight percent), here and throughout the description unless otherwise defined, refers to the relative weight of the respective component based on the overall weight of the formulation.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the term "diaper" refers to an absorbent article generally worn by infants and incontinent persons about the lower torso.

By "stretch", it is meant that the material has the ability to extend beyond its original size in at least one dimension when subjected to a tensile force (i.e., tension) applied in the direction of that dimension. "Stretch" may be unidirectional, bi-directional, or multi-directional. The specific "stretch" properties of a material may vary along any of the stretch vectors. As used herein, stretch includes both plastic and elastic deformation.

The term "elastic" or "elastomeric" as used herein refers to any material that upon application of a biasing force, can stretch to an elongated size of at least about 125 percent of its relaxed, original size, without rupture or breakage, and upon release of the applied force, recovers at least about 40% of its elongation, preferably recovers at least 60% of its original size, more preferably recovers at least about 80%, most preferably recovers about 100% of its original size.

The terms "belly section" and "front belly section" are used herein as synonyms and refer to the area of the absorbent article which is contact with the belly of the wearer when the absorbent article is worn.

The terms "back section" and "rear back section" are used herein as synonyms and refer to the area of the absorbent article which is contact with the back of the wearer when the absorbent article is worn.

As used herein, the terms "elastic resistance" and "restoring force" describe an elastic force that tends to resist an applied tensile force causing a material provided therewith to tend to contract to an untensioned configuration in response to a stretching force.

"Longitudinal" is a direction running parallel to the maximum linear dimension of the article and includes directions within ±45 of the longitudinal direction. The "lateral" or "transverse" direction is orthogonal to the longitudinal direction.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

The term "non-woven" as used herein refers to a manufactured sheet, web, or batt directionally or randomly oriented fibers bonded by friction and/or cohesion and/or adhesion, excluding paper and products that are woven, knitted, tufted stitchbonded incorporating binding yarns or filaments, or felted by wet-milling, whether or not additionally needled. The fibers may be of natural or man-made origin. They may also be staple or continuous filaments or be formed in situ.

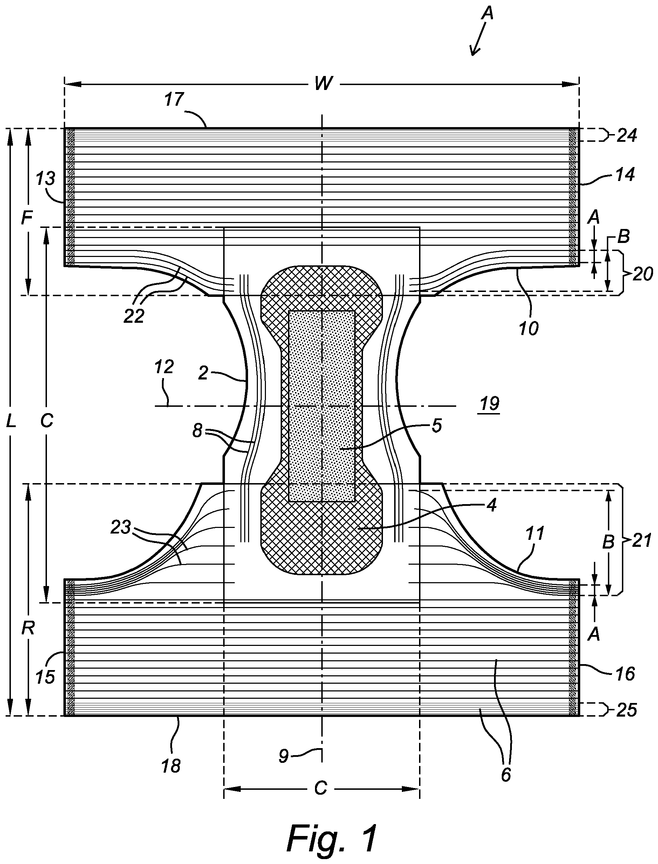

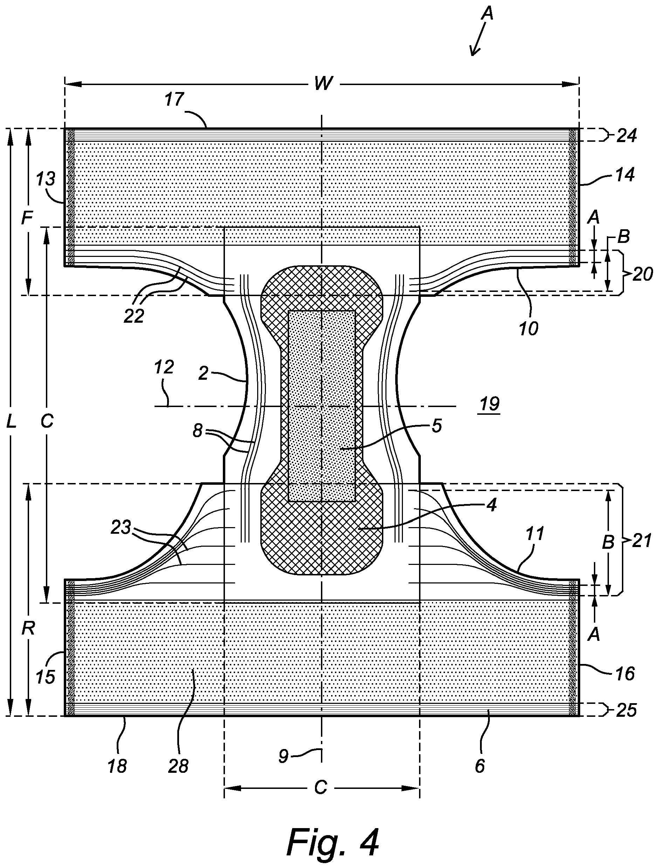

In a preferred embodiment, the present invention provides an absorbent article suitable to be worn about the lower torso of a wearer and comprising a front belly section **F** and a rear back section **R,** each of said section is provided with longitudinal edges **13, 14, 15, 16** and a transversal edge **17, 18** (**Fig. 1**). The article **A** further comprises a crotch section **C** extending between the front belly section **F** and the rear back section **R** and is permanently attached to the front belly section **F** and the rear back section **R.** The crotch section **C** comprises an absorbent body **4** and extends in the direction of a longitudinal center axis **9** of the absorbent article **A** (**Fig. 1**). The crotch section **C,** the front belly section **F** and the rear back section **R** define leg openings **19** of the absorbent article **A.** The front belly section **F** and the rear back section **R** have an edge contour **10, 11** which extends starting from the longitudinal edges **13, 14, 15, 16** towards a transverse center axis **12** of the crotch section **C** (**Fig. 1**). The front belly section **F** and the rear back section **R** comprise first elastification means **6,** and, in an area **20, 21** of the front belly section **F** and the rear back section **R** on the crotch side facing the leg openings **19,** second elastification means **22, 23** are provided. Said second elastification means **22, 23** extend starting from the longitudinal edges **13, 14, 15, 16** towards the longitudinal center axis **9** of the absorbent article **A** in a curved shape and are fanning out with increasing separation from each other in the rear back section **R** and with equal separation from each other and parallel to each other in the front belly section **F** (**Fig. 1**). In a preferred embodiment, the second elastification means **22** of the front belly section **F** extend starting from the longitudinal edges **13, 14** of said front belly section **F** towards the longitudinal center axis **9** of the absorbent article **A** in a curved shape. Said second elastification means **22** extends essentially perpendicular to the longitudinal edges **13, 14** of the front belly section **F** and parallel to each other. The second elastification means **22** continues extension in a curved manner and also parallel to each other towards the longitudinal center axis **9** of the absorbent article **A** as shown in **Fig. 1** and **Fig. 4**. Preferably, said second elastification means continues extension in the crotch section C wherein they are preferably extending essentially perpendicular to the longitudinal center axis **9** of the absorbent article **A**.

In a preferred embodiment, the present invention provides a disposable absorbent article **A,** suitable to be worn about the lower torso of a wearer, comprising a front belly section F and a rear back section **R,** each of said section is provided with longitudinal edges **13, 14, 15, 16** and a transversal edge **17, 18**; said article **A** further comprises a crotch section **C** extending between the front belly section F and the rear back section **R** and is permanently attached to the front belly section **F** and the rear back section **R,** said crotch section **C** comprises an absorbent body **4** and extends in the direction of a longitudinal center axis **9** of the absorbent article **A**; the crotch section **C,** the front belly section **F** and the rear back section **R** define leg openings **19** of the absorbent article **A** and the front belly section **F** and the rear back section **R** have an edge contour **10, 11** which extends starting from the longitudinal edges **13, 14, 15, 16** towards a transverse center axis **12** of the crotch section **C**; wherein said front belly section **F** and rear back section **R** comprise first elastification means **6, 28,** and, in an area **20, 21** of the front belly section **F** and the rear back section **R** on the crotch side facing the leg openings **19,** second elastification means **22, 23** are provided, said second elastification means **22, 23** extend starting from longitudinal edges **13, 14, 15, 16** towards the longitudinal center axis **9** of the absorbent article **A** in a curved shape. The front belly section **F** and/or the rear back section **R** further comprise an elastified material **28** provided on 1% to 65%, preferably 20% to 60%, more preferably on 40 to 55%, most preferably on at least 50% of the surface area of said front belly section **F** and/or the rear back section **R**.

In a preferred embodiment, the present invention provides a disposable absorbent article **A,** suitable to be worn about the lower torso of a wearer, comprising a front belly section **F** and a rear back section **R,** each of said section is provided with longitudinal edges **13, 14, 15, 16** and a transversal edge **17, 18**; said article **A** further comprises a crotch section **C** extending between the front belly section **F** and the rear back section **R** and is permanently attached to the front belly section **F** and the rear back section **R,** said crotch section **C** comprises an absorbent body **4** and extends in the direction of a longitudinal center axis **9** of the absorbent article **A**; the crotch section **C,** the front belly section **F** and the rear back section **R** define leg openings **19** of the absorbent article **A** and the front belly section **F** and the rear back section **R** have an edge contour **10, 11** which extends starting from the longitudinal edges **13, 14, 15, 16** towards a transverse center axis **12** of the crotch section **C**; wherein said front belly section **F** and rear back section **R** comprise first elastification means **6, 28,** and, in an area **20, 21** of the front belly section **F** and the rear back section **R** on the crotch side facing the leg openings **19,** second elastification means **22, 23** are provided, said second elastification means **22, 23** extend starting from longitudinal edges **13, 14, 15, 16** towards the longitudinal center axis **9** of the absorbent article **A** in a curved shape. The surface proportion of the crotch section **C** with respect to the entire surface of the absorbent article **A** is of from 25 to 55%, said crotch section overlaps at least 12% of the surface of the front belly section **F** and at least 20% of the surface of the rear back section **R** in an extended state of the absorbent article and wherein the absorbent body **4** overlaps at least 5% of the surface of the front belly section **F** and/or at least 10% of the surface of the rear back section **R**. This design is advantageous as the absorbent article can be a "low waist" article which provides comfort to the wearer.

In a preferred embodiment, the front belly section **F,** the rear back section **R** and the crotch section **C** may be independently produced, i.e. a front belly section **F,** a rear back section **R,** and a crotch section **C** which is disposed there between and comprises an absorbent body **4**. In a further preferred embodiment, for assembling the absorbent article, the crotch section **C** overlaps a substantial surface portion of the front belly section **F** and also of the rear back section **R.**

The front belly section **F** can be divided into an area **20** on the crotch side facing the leg openings and a hip side area which extends between the transversal edge of the section **F** and the area **20**. A corresponding subdivision is provided in the rear back section **R,** i.e. also into a hip side area and an area **21** on the crotch side facing the leg openings.

The first elastification means **6** are provided in the hip side area of the belly section **F** and/or in the hip side area of the back section **R**. In a preferred embodiment, said first elastification means **6** may be thread-like elastification means, such as Lycra threads, which are pretensioned and connected to the sheet materials of the belly section **F** and the back section **R** in a so-called stretch-bond method. These first elastification means **6** extend from one longitudinal edge to the other longitudinal edge of each section. In a preferred embodiment, the first elastification means 6 extend, at a separation from each other and parallel to each other and parallel to the transversal edge 17, 18 of the front belly section and the rear back section thereby extensively elasticising said sections (Fig. 1).

In a preferred embodiment, the first elastification means 6 of the back section R and/or the front belly section F have a minimum separation of 3 to 15 mm, preferably 4 to 14 mm, more preferably 5 to 12 mm, most preferably 6 to 11 mm from each other. By separation, it is meant the separation of directly neighboring elastification means.

In a preferred embodiment, in an area 24, 25 proximal to the transversal edge 17, 18 of the front belly section F and/or of the rear back section R, the first elastification means 6 are extending at a separation from each other which is at least 50% smaller than the separation of the first elastification means in the remaining area of said front belly section F and/or the rear back section R. This ensures a better fitting of the absorbent article around the wearer's waist. Said area 24, 25 extends in the longitudinal direction from the transversal edge towards the central transversal axis 12. Preferably said area extends over 10 to 30 mm, preferably 11 to 25, more preferably over 12 to 20 mm, most preferably about 15 mm. Preferably, in said area 24, 25, the first elastification means 6 of the back section R and/or the front belly section F have a minimum separation of 0.5 to 5 mm, preferably 1 to 4 mm, more about 3 mm from each other. By separation, it is meant the separation of directly neighboring elastification means.

In a preferred embodiment, the front belly section F and/or the rear back section R further comprise an elastified material 28 provided on 1% to 65%, preferably 20% to 60%, more preferably on 40 to 55%, most preferably on at least 50% of the surface area of said front belly section F and/or the rear back section R (Fig. 4). In a further preferred embodiment, said elastified material 28 is positioned between the area comprising the first elastification means 6 which is proximal to the transversal edge 17, 18 of the front belly section F and/or of the rear back section R and the area 20 or 21 on the crotch side facing the leg openings. In a preferred embodiment said elastified material extends in the longitudinal direction over at least 140 to 160 mm, preferably 145 to 150 mm, more preferably over about 148 mm of the front belly section F and/or the rear back section R. It is to be understood that all the features of the first embodiment of the absorbent article described herein and/or with reference to Fig. 1 to 3 are also applicable for the second embodiment of the absorbent article represented in Fig. 4 to 6.

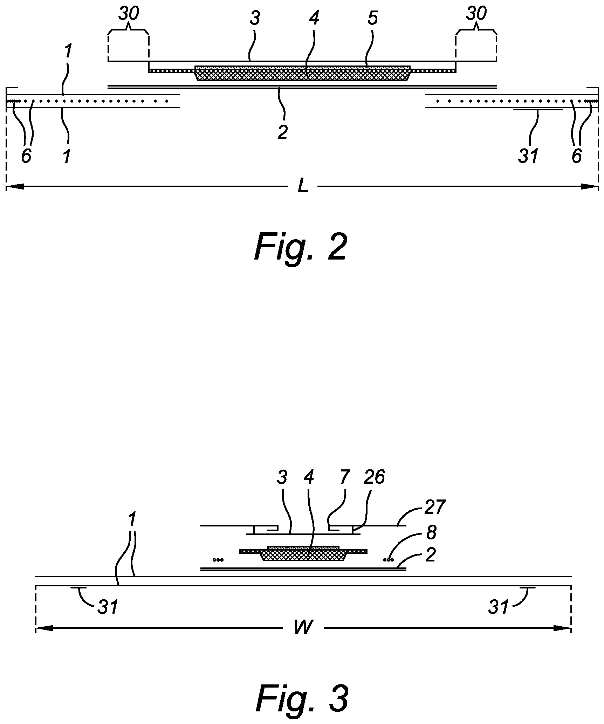

The area 20 of the belly section F or 21 of the back section R on the crotch side facing the leg openings 19 has an edge contour 10 or 11 which extends towards a transverse center axis 12 of the crotch section C. This edge contour 10, 11 is curved as shown in Fig. 1 and 2

and therefore suited for delimiting the leg openings 19. The area 20 or 21 on the crotch side facing the leg openings realizes a relatively large overlapping area between the crotch section C and the belly section F or back section R, which is essential in view of a tear-resistant connection between the crotch section C and the belly section F or back section R.

In a preferred embodiment, the crotch section C overlaps at least 12%, preferably at least 15%, more preferably at least 20%, most preferably at least 25% of the surface of the front belly section F and at least 20%, preferably at least 25%, more preferably at least 30%, most preferably at least 35% of the surface of the rear back section R in an extended state of the absorbent article. It is to be understood that by extended state, reference is made to the absorbent article when flattened on a surface as represented in Fig. 1 and 2.

In a preferred embodiment, the crotch section C overlaps at most 50%, preferably at most 40%, more preferably at most 35%, most preferably at most 30% of the surface of the front belly section F and at most 50%, preferably at most 45%, more preferably at most 40%, most preferably at most 30% of the surface of the rear back section R in an extended state of the absorbent article.

In a preferred embodiment, the absorbent body 4 overlaps at least 5%, preferably at least 8%, more preferably at least 10% of the surface of the front belly section F and/or at least 10%, preferably at least 12%, more preferably at least 15% of the surface of the rear back section R. The absorbent body 4 overlaps at most 20% of the surface of the front belly section F and/or at most 20% of the surface of the rear back section R.

The areas 20, 21 of the belly section F or the back section R on the crotch side facing the leg openings 19 is also elasticised. Second elastification means 22 and 23 are provided therein. The second elastification means 22, 23 extend in each case towards a longitudinal center axis 9 of the absorbent article and starting from the longitudinal edge 13, 14, 15, 16 until reaching the area of the absorbent article wherein the crotch section C overlaps with the front belly section F or with the rear back section R. As is illustrated in Fig. 1 and 4, the second elastification means 22, 23 extend in a curved shape and are fanning out with increasing separation from each other in the rear back section R and with equal separation from each other and parallel to each other in the front belly section F.

The second elastification means 23 of the back section R e.g. have a minimum separation of from 1 to 10 mm, preferably of from 2 to 9 mm, more preferably of from 3 to 8 mm from each other at the longitudinal edge 15, 16 of said section and a maximum separation of 7 to 35 mm on the crotch section C overlapping with the back section R of the absorbent article. By separation, it is meant the separation of directly neighboring elastification means.

In a preferred embodiment, a minimum separation between the second elastification means **22** of the front belly section **F** is 3 to 20 mm, preferably 4 to 15 mm, more preferably 5 to 10 mm, most preferably 6 to 8 mm. By separation, it is meant the separation of directly neighboring elastification means.

In a preferred embodiment, the fanning-out degree FO of the second elastification means **22, 23** is as follows:

$$FO = (A-B)/B*100\%$$

Wherein,
**A** is the distance separating, in the longitudinal direction **9**, the second elastification means **22, 23** which is the most proximal to the transversal edge **17, 18** from the second elastification means **22, 23** which is the most proximal to the edge contour **10, 11** i.e. not the separation between directly neighboring elastification means. **A,** shown in **Fig. 1** and **Fig. 4,** is then the separation at the longitudinal edge **13, 14, 15, 16** of the front belly section or the rear back section.

**B** is the distance separating, in the longitudinal direction **9**, the second elastification means **22, 23** which is the most proximal to the transversal edge **17, 18** from the projection of the second elastification means **22, 23** which is the most proximal to the edge contour **10, 11**, wherein said projection is parallel to the transversal edge **17, 18** i.e. not the separation between directly neighboring elastification means (**Fig. 1** and **Fig. 4**).

In a preferred embodiment, the fanning-out degree of the second elastification means **23** in the rear back section **R** is larger than the fanning-out degree of the second elastification means **22** in the front belly section **F.**

In a preferred embodiment, the surface proportion of the crotch section **C** with respect to the entire surface of the absorbent article **A** is of from 25 to 55%. In a preferred embodiment, the separation between the front belly section **F** and the rear back section **R** in the longitudinal direction **9** of the absorbent article is of from 200 to 500 mm, preferably from 220 to 450 mm, more preferably from 250 to 400 mm, most preferably from 280 to 380 mm.

In a preferred embodiment, the separation between the most proximal second elastification means **22, 23** to the crotch area **C,** and the edge contour **10, 11**, defining the leg openings **19** of the belly section **F** and the back section **R** on the crotch side facing the leg openings is of from 2 to 40 mm, preferably of from 3 to 30 mm, more preferably of from 4 to 15 mm, most preferably of from 5 to 12 mm.

In a preferred embodiment, the crotch section **C** comprises a liquid-impermeable back sheet material **2,** which may be formed, in particular, by a breathable, but liquid-tight foil material, and a top sheet material **3** that is preferably based on a non-woven material. The absorbent body **4** is disposed between the back sheet material and the top sheet material (shown in **Fig. 2, 3**). Preferably, the back sheet material **2** and/or the top sheet material **3** forms a projection **30** that extends past the absorbent body **4** in the transverse direction **12**. The projection **30** may extend past the absorbent body **4** in the transverse direction **12** on both sides of said absorbent body **4,** i.e. on the left-hand and right-hand side added together, amounts to at least 25%, preferably 30 to 45%, more preferably 35 to 45%, with respect to the maximum width of the crotch section (i.e. with respect to the maximum extension of the crotch section in the transverse direction).

By top sheet reference is made to a liquid permeable material sheet which in use is placed in direct contact with the skin of the wearer. The top sheet can comprise a nonwoven material, e.g. spunbond, meltblown, carded, hydroentangled, wetlaid etc. Suitable nonwoven materials can be composed of man-made fibers, such as polyester, polyethylene, polypropylene, viscose, rayon etc. or natural fibers, such as wood pulp or cotton fibers, or from a mixture of natural and man-made fibers. The top sheet material may further be composed of two fibers, which may be bonded to each other in a bonding pattern, as e.g. disclosed in EP 1 035 818. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid.

By back sheet reference is made to a material forming the outer cover of the absorbent article. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapor to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The "absorbent body" is the absorbent structure disposed between the top sheet and the back sheet of the absorbent article in at least the crotch region thereof. The absorbent material can be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbent polymers in an absorbent material. Superabsorbent polymers are water-swellable, water-insoluble organic or inorganic materials capable of absorbing at least about times their own weight of an aqueous solution containing 0.9 weight percent of sodium chloride. Organic materials suitable for use as superabsorbent materials can include natural materials such as polysaccharides, polypeptides and the like, as well as synthetic materials such as synthetic hydrogel polymers. Such hydrogel polymers include, for example, alkali metal salts of polyacrylic acids, polyacrylamides, polyvinyl alcohol, polyacrylates, polyvinyl pyridines, and the like. Other suitable polymers include hydrolyzed acrylonitrile grafted starch, acrylic acid grafted starch, and isobutylene maleic anhydride copolymers and mixtures thereof. The hydrogel polymers are preferably lightly cross-linked to render the material substantially water insoluble. Preferred superabsorbent materials are further surface cross-linked so that the outer surface or shell of the superabsorbent particle, fiber, flake, sphere, etc. possesses a higher crosslink density than the inner portion of the superabsorbent. The superabsorbent materials may be in any form which is suitable for use in absorbent composites including particles, fibers, flakes, spheres, and the like.

In a preferred embodiment, the absorbent body **4** is at least partially covered by a high loft material **5,** i.e. low density, thick or bulky fabric such as high loft nonwovens. This is advantageous as it allows reaching superior dryness through the key performance of the high loft martial: fast acquisition of the fluid, wide spreading of the liquid and enhanced buffer function. Furthermore, the high loft material works as barrier between the wet mass and the user's skin.

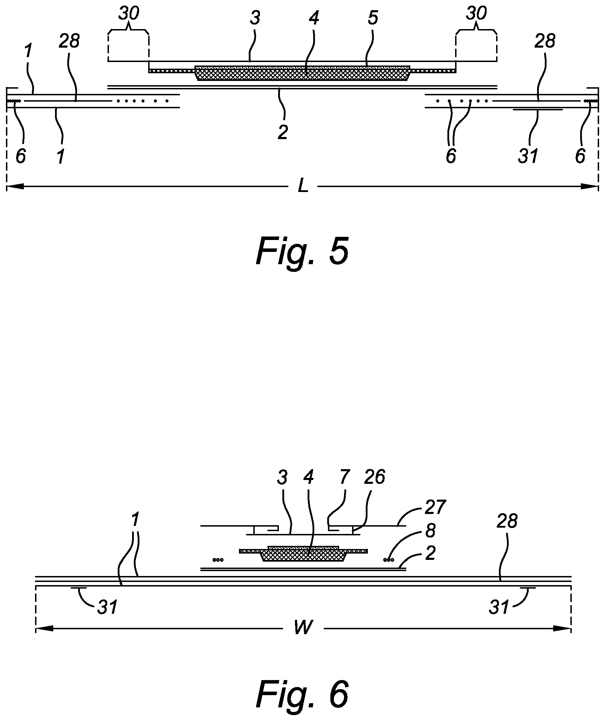

In a preferred embodiment, the back sheet of the absorbent article rear back section is provided with at least one releasable fastening means **31** (**Fig. 3** and **Fig. 6**). Said releasable fastening are used for fastening the absorbent article around a wearer's waist by attaching the back section **R** to the front section **F** of the absorbent article. The fastening means may be in the form of mechanical fasteners of the hook and loop type, or adhesive tape fasteners. In a preferred embodiment, the absorbent article is provided with at least one landing zone which is provided in the front section. Said landing zone indicates the position in which each fastening means should be attached to the front section of the absorbent article. This ensures an optimal and symmetric attachment of the fastening means to the front section of the article.

In a preferred embodiment, the crotch section is provided with at least one upstanding cuff **27** on each side of the absorbent body **4,** which extends in the longitudinal direction **9**. Said upstanding cuffs **27** are preferably formed from a hydrophobic, preferably liquid-impermeable non-woven material, which preferably extends in the transverse direction **12** to the lateral longitudinal edges of the crotch section **C**. The upstanding cuffs **27** are provided, at their ends which are proximal to the central longitudinal axis **9,** with further elastification means **7** (**Fig. 3**). Said elastification means **7** lift the upstanding cuffs **27** during use of the absorbent article against the skin surface of the user. The upstanding cuffs **27** are fixed via fixations **26** to the top sheet **3.** Preferably, the crotch section overlaps an area of the front belly section and an area of the rear back section in an extended state of the absorbent article.

In a preferred embodiment, the leg elastification means **8** of the crotch section **C** extend outside and at a given separation from the absorbent body **4** along the leg opening **19**. This configuration is advantageous as it prevents the absorbent body from any additional extension or torsional forces which could impair its absorption properties thereby leading to a liquid-tight leg termination which is largely independent of the absorbent body.

In a preferred embodiment, the leg elastification means **8** of the crotch section **C** terminate in the longitudinal direction **9** in the front belly section **F** and in the rear back section **R**. Preferably, the crotch section **C** comprises leg elastification means **8** which extend outside of the absorbent body **4** along the leg opening **19** and terminate in the longitudinal direction **9** in an area of overlap between said crotch section **C** and the front belly section **F** and in an area of overlap between said crotch section **C** and in the rear back section **R.** In a further preferred embodiment, the leg elastification means **8** terminate in the longitudinal direction **9** in the area **20, 21** of the front belly section **F** and the rear back section **R** wherein the second elastification means **22, 23** are provided.

In a preferred embodiment, thread-shaped or band-shaped elastification means, such as rubber or polyether polyurethane or polyester polyurethane threads, preferably elastic threads such as Lycra(R) or Spandex(R) threads, are used as leg elastification means. The leg elastification means preferably have a thickness of 300 to 1500 dtex, in particular, 500 to 1200 dtex, moreover, in particular 500 to 900 dtex.

It is particularly advantageous for the leg elastification means **8** to terminate in the longitudinal direction **9** in the area **20, 21** of the front belly section **F** and the rear back section **R** wherein the second elastification means **22, 23** are provided. The restoring force of said leg elastification means 8 will add to the restoring force of the second elastification means **22, 23.** Said restoring forces will have different directions which improves the fit of the absorbent article to the wearer's body. In addition, said restoring forces prevent excessive pressure and/or cutting of the wearer's skin which happens when only restoring forces of the second elastification means are present in the area **20, 21** on the crotch side facing the leg openings.

In order to determine the restoring forces, the areas of the absorbent article to be measured can be fixedly clamped directly and quasi non-destructively between two clamping jaws of defined identical clamping jaw width, and the restoring forces can be determined under a defined extension, which simulates the state of wear, of the areas to be measured by, in particular, 30% or 50% or 80% of the initial length (of the clamping jaw separation during fixing of the area to be measured in the non-stretched state). The clamping jaws should fix as many as possible, however, at least two adjacent elastification means of the area to be measured, and be substantially oriented perpendicularly to the extension of the elastification means, such that the clamps are extended substantially in the direction of extension of the elastification means.

In a preferred embodiment, the liquid-impermeable back sheet material **2** of the crotch section **C** is provided with a reinforcing coating over at least the area of overlap of the crotch section **C** with the front belly section **F** and/or with the rear back section **R**. The reinforcing coating terminates in the longitudinal direction **9** in the crotch section **C**. In a preferred embodiment, the reinforcing coating extends in the longitudinal direction **9** from the overlap area of the belly section **F** with the crotch section **C** to the crotch section **C**. Similarly, the reinforcing coating extends in the longitudinal direction **9** from the overlap area of the back section **R** with the crotch section **C** to the crotch section **C**. This is advantageous as it strengthen the joining of the crotch section **C** to the belly section **F** or the back section **R**. In a preferred embodiment, the reinforcing coating consists of a non-woven material, in particular, of a spunbonded non-woven material of polypropylene.

Although the present invention has been described with reference to preferred embodiments thereof, many modifications and alternations may be made by a person having ordinary skill in the art without departing from the scope of this invention which is defined by the appended claims.

**Claims**

1.  A disposable absorbent article (A), suitable to be worn about the lower torso of a wearer, comprising a front belly section (F) and a rear back section (R), each of said section is provided with longitudinal edges (13, 14, 15, 16) and a transversal edge (17, 18); said article (A) further comprises a crotch section (C) extending between the front belly section (F) and the rear back section (R) and is permanently attached to the front belly section (F) and the rear back section (R), said crotch section (C) comprises an absorbent body (4) and extends in the direction of a longitudinal center axis (9) of the absorbent article (A); the crotch section (C), the front belly section (F) and the rear back section (R) define leg openings (19) of the absorbent article (A) and the front belly section (F) and the rear back section (R) have an edge contour (10, 11) which extends starting from the longitudinal edges (13, 14, 15, 16) towards a transverse center axis (12) of the crotch section (C); wherein said front belly section (F) and rear back section (R) comprise first elastification means (6, 28), and, in an area (20, 21)

of the front belly section (F) and the rear back section (R) on the crotch side facing the leg openings (19), second elastification means (22, 23) are provided, said second elastification means (22, 23) extend starting from longitudinal edges (13, 14, 15, 16) towards the longitudinal center axis (9) of the absorbent article (A) in a curved shape and are fanning out with increasing separation from each other in the rear back section (R) and with equal separation from each other and parallel to each other in the front belly section (F).

2.  Absorbent article according to claim 1, wherein the crotch section (C) comprises leg elastification means (8) which extend outside of the absorbent body (4) along the leg opening (19) and terminate in the longitudinal direction (9) in an area of overlap between said crotch section (C) and the front belly section (F) and in an area of overlap between said crotch section (C) and in the rear back section (R).

3.  Absorbent article according to any one or more of the preceding claims, wherein the leg elastification means (8) terminate in the longitudinal direction (9) in the area (20, 21) of the front belly section (F) and the rear back section (R) wherein second elastification means (22, 23) are provided.

4.  Absorbent article (A) according to any one or more of the preceding claims, wherein the first elastification means (6) extend, at a separation from each other and parallel to each other and parallel to the transversal edge (17, 18) thereby extensively elasticising the front belly section (F) and the rear back section (R).

5.  Absorbent article (A) according to any one or more of the preceding claims, wherein the front belly section (F) and/or the rear back section (R) further comprise an elastified material (28) provided on 1% to 65% of the surface area of said front belly section (F) and/or the rear back section (R).

6.  Absorbent article according to any one or more of the preceding claims, wherein the fanning-out degree of the second elastification means (23) in the rear back section (R) is larger than the fanning-out degree of the second elastification means (22) in the front belly section (R).

7.  Absorbent article according to any one or more of the preceding claims, wherein the separation between the second elastification means (22) of the front belly section (F) is 3 to 20 mm, preferably 4 to 15 mm, more preferably 5 to 10 mm, most preferably 6 to 8 mm.

8.  Absorbent article according to any one or more of the preceding claims, wherein the crotch section (C) comprises a liquid-impermeable back sheet material (2) and a top sheet material (3) between which the absorbent body (4) is disposed, and the back sheet material (2) and/or the top sheet material (3) form a projection (30) that extends past the absorbent body (4) in the transverse direction (12) and on both sides of said absorbent body (4).

9. Absorbent article according to any one or more of the preceding claims, wherein the absorbent body (4) is at least partially covered by a high loft material (5).

10. Absorbent article according to any one or more of the preceding claims, wherein the surface proportion of the crotch section (C) with respect to the entire surface of the absorbent article (A) is of from 25 to 55%.

11. Absorbent article according to any one or more of the preceding claims, wherein the crotch section (C) overlaps at least 12% of the surface of the front belly section (F) and at least 20% of the surface of the rear back section (R) in an extended state of the absorbent article.

12. Absorbent article according to any one or more of the preceding claims, wherein the absorbent body (4) overlaps at least 5% of the surface of the front belly section (F) and/or at least 10% of the surface of the rear back section (R).

13. Absorbent article according to any one or more of the preceding claims, wherein the front belly section (F) and/or the rear back section (R) further comprise an elastified material (28) provided on at least 1% to 65% of the surface area of said front belly section (F) and/or the rear back section (R).

14. Absorbent article according to any one or more of the preceding claims, wherein the surface proportion of the crotch section (C) with respect to the entire surface of the absorbent article (A) is of from 25 to 55%, said crotch section overlaps at least 12% of the surface of the front belly section (F) and at least 20% of the surface of the rear back section (R) in an extended state of the absorbent article and wherein the absorbent body (4) overlaps at least 5% of the surface of the front belly section (F) and/or at least 10% of the surface of the rear back section (R).

**Patentansprüche**

1. Saugfähiger Einwegartikel (A), der geeignet ist, um um den unteren Torso eines Trägers getragen zu werden, umfassend einen vorderen Bauchabschnitt (F) und einen hinteren Rückenabschnitt (R), wobei jeder der Abschnitte mit Längsrändern (13, 14, 15, 16) und einem Querrand (17, 18) ausgestattet ist; wobei der Artikel (A) weiter einen Schrittabschnitt (C) umfasst, der sich zwischen vom vorderen Bauchabschnitt (F) und dem hinteren Rückenabschnitt (R) erstreckt und permanent an den vorderen Bauchabschnitt (F) und den hinteren Rückenabschnitt (R) befestigt ist, wobei der Schrittabschnitt (C) einen saugfähigen Körper (4) umfasst und sich in der Richtung einer Längsmittelachse (9) des saugfähigen Artikels (A) erstreckt; wobei der Schrittabschnitt (C), der vordere Bauchabschnitt (F) und der hintere Rückenabnbschnitt (R) Fußöffnungen (19) des saugfähigen Artikels (A) definieren und der vordere Bauchabschnitt (F) und der hintere Rückenabschnitt (R) einen Randumfang (10, 11) aufweisen, der sich ausgehend von den Längsrändern (13, 14, 15, 16) hin zu einer Quermittelachse (12) des Schrittabschnitts (C) erstreckt; wobei der vordere Bauchabschnitt (F) und der hintere Rückenabschnitt (R) erste Elastifizierungsmittel (6, 28) umfassen, und, in einem Bereich (20, 21) des vorderen Bauchabschnitts (F) und des hinteren Rückenabschnitts (R) auf der Schrittseite, die den Fußöffnungen (19) gegenüber liegt, zweite Elastifizierungsmittel (22, 23) bereitgestellt sind, wobei sich die zweiten Elastifizierungsmittel (22, 23) ausgehend von den Längsrändern (13, 14, 15, 16) hin zur Längsmittelachse (9) des saugfähigen Artikels (A) in einer gekrümmten Form erstrecken und mit zunehmender Trennung voneinander im hinteren Rückenabschnitt (R) und mit gleicher Trennung voneinander und parallel zueinander im vorderen Bauchabschnitt (F) auffächern.

2. Saugfähiger Artikel nach Anspruch 1, wobei der Schrittabschnitt (C) Fußelastifizierungsmittel (8) umfasst, die sich außerhalb des saugfähigen Körpers (4) entlang der Fußöffnung (19) erstrecken und in der Längsrichtung (9) in einem Bereich des Überlappens zwischen dem Schrittabschnitt (C) und dem vorderen Bauchabschnitt (F) in einem Bereich des Überlappens zwischen dem Schrittabschnitt (C) und dem hinteren Rückenabschnitt (R) enden.

3. Saugfähiger Artikel nach einem oder mehreren der vorhergehenden Ansprüche, wobei die Fußelastifizierungsmittel (8) in der Längsrichtung (9) im Bereich (20, 21) des vorderen Bauchabschnitts (F) und des hinteren Rückenbschnitts (R) enden, wo zweite Elastifizierungsmittel (22, 23) bereitgestellt sind.

4. Saugfähiger Artikel (A) nach einem oder mehreren der vorhergehenden Ansprüche, wobei sich die ersten Elastifizierungsmittel (6) mit einer Trennung voneinander und parallel zueinander und parallel zum Querrand (17, 18) erstrecken, wodurch der vordere Bauchabschnitt (F) und der hintere Rückenabschnitt (R) weitgehend elastifiziert werden.

5. Saugfähiger Artikel (A) nach einem oder mehreren der vorhergehenden Ansprüche, wobei der vordere Bauchabschnitt (F) und/oder der hintere Rückenabschnitt (R) weiter ein elastifiziertes Material (28) umfassen, das auf 1 % bis 65 % des Oberflächenbereichs des vorderen Bauchabschnitts (F) und/oder des hinteren Rückenabschnitts (R) bereitgestellt ist.

6. Saugfähiger Artikel nach einem oder mehreren der vorhergehenden Ansprüche, wobei der Grad der Auffächerung der zweiten Elastifizierungsmittel (23) im hinteren Rückenabschnitt (R) größer als der Grad der Auffächerung der zweiten Elastifizierungsmittel (22) im vorderen Bauchabschnitt (R) ist.

7. Saugfähiger Artikel nach einem oder mehreren der vorhergehenden Ansprüche, wobei die Trennung zwischen den zweiten Elastifizierungsmitteln (22) und dem vorderen Bauchabschnitt (F) 3 bis 20 mm, vorzugsweise 4 bis 15 mm, insbesondere 5 bis 10 mm, am meisten bevorzugt 6 bis 8 mm beträgt.

8. Saugfähiger Artikel nach einem oder mehreren der vorhergehenden Ansprüche, wobei der Schrittabschnitt (C) ein Flüssigkeits-undurchlässiges Rückseiten-Folienmaterial (2) und ein oberes Folienmaterial (3) umfasst, zwischen denen der saugfähige Körper (4) angeordnet ist, und das hintere Folienmaterial (2) und/oder das obere Folienmaterial (3) einen Vorsprung (30) bilden, der sich am saugfähigen Körper (4) vorbei in der Querrichtung (12) und auf beiden Seiten des saugfähigen Körpers (4) erstreckt.

9. Saugfähiger Artikel nach einem oder mehreren der vorhergehenden Ansprüche, wobei der saugfähige Körper (4) mindestens teilweise von einem hochporösen Material (5) bedeckt ist.

10. Saugfähiger Artikel nach einem oder mehreren der vorhergehenden Ansprüche, wobei der Oberflächenteil des Schrittabschnitts (C) mit Bezug auf die gesamte Oberfläche des saugfähigen Artikels (A) von 25 bis 55 % beträgt.

11. Saugfähiger Artikel nach einem oder mehreren der vorhergehenden Ansprüche, wobei der Schrittabschnitt (C) mindestens 12 % der Oberfläche des vorderen Bauchabschnitts (F) und mindestens 20 % der Oberfläche des hinteren Rückenabschnitts (R) in einem erweiterten Zustand des saugfähigen Artikels umfasst.

12. Saugfähiger Artikel nach einem oder mehreren der vorhergehenden Ansprüche, wobei der saugfähige Körper (4) mindestens 5 % der Oberfläche des vorderen Bauchabschnitts (F) und mindestens 10 % der Oberfläche des hinteren Rückenabschnitts (R) überlappt.

13. Saugfähiger Artikel nach einem oder mehreren der vorhergehenden Ansprüche, wobei der vordere Bauchabschnitt (F) und/oder der hintere Rückenabschnitt (R) weiter eine elastifiziertes Material (28) umfassen, das auf 1 % bis 65 % des Oberflächenbereichs des vorderen Bauchabschnitts (F) und/oder des hinteren Rückenabschnitts (R) bereitgestellt ist.

14. Saugfähiger Artikel nach einem oder mehreren der vorhergehenden Ansprüche, wobei der Oberflächenteil des Schrittabschnitts (C) mit Bezug auf die gesamte Oberfläche des saugfähigen Artikels (A) von 25 bis 55 % beträgt, der Schrittabschnitt (C) mindestens 12 % der Oberfläche des vorderen Bauchabschnitts (F) und mindestens 20 % der Oberfläche des hinteren Rückenabschnitts (R) in einem erweiterten Zustand des saugfähigen Artikels umfasst, und wobei der saugfähige Körper (4) mindestens 5 % der Oberfläche des vorderen Bauchabschnitts (F) und/oder mindestens 10 % der Oberfläche des hinteren Rückenabschnitts (R) überlappt.

## Revendications

1. Article absorbant jetable (A), adapté pour être porté autour du torse inférieur d'une personne, comprenant une section avant d'abdomen (F) et une section arrière de dos (R), chacune desdites sections étant munie de bords longitudinaux (13, 14, 15, 16) et d'un bord transversal (17, 18) ; ledit article (A) comprenant en outre une section d'entrejambe (C) s'étendant entre la section avant d'abdomen (F) et la section arrière de dos (R) et étant reliée de manière permanente à la section avant d'abdomen (F) et à la section arrière de dos (R), ladite section d'entrejambe (C) comprenant un corps absorbant (4) et s'étendant dans la direction d'un axe central longitudinal (9) de l'article absorbant (A) ; la section d'entrejambe (C), la section avant d'abdomen (F) et la section arrière de dos (R) définissant des ouvertures de jambes (19) de l'article absorbant (A) et la section avant d'abdomen (F) et la section arrière de dos (R) ayant un contour de bord (10, 11) qui s'étend des bords longitudinaux (13, 14, 15, 16) vers un axe central transversal (12) de la section d'entrejambe (C) ; dans lequel ladite section avant d'abdomen (F) et ladite section arrière de dos (R) comprennent des premiers moyens d'élastification (6, 28), et, dans une zone (20, 21) de la section avant d'abdomen (F) et la section arrière de dos (R), sur le côté entrejambe tourné vers les ouvertures de jambes (19), des seconds moyens d'élastification (22, 23) sont prévus, lesdits seconds moyens d'élastification (22, 23) s'étendant entre les bords longitudinaux (13, 14, 15, 16) et l'axe central longitudinal (9) de l'article absorbant (A) en forme de courbe, et se répartissant en se séparant de plus en plus les uns des autres dans la section arrière de dos (R), et en se séparant de manière égale les uns des autres et parallèlement les uns aux autres dans la section avant d'abdomen (F).

2. Article absorbant selon la revendication 1, dans lequel la section d'entrejambe (C) comprend des moyens d'élastification de jambe (8) qui s'étendent à l'extérieur de l'article absorbant (4) le long de l'ouverture de jambe (19) et se terminent dans la direction longitudinale (9) dans une zone de chevauchement entre ladite section d'entrejambe (C) et la section avant d'abdomen (F) et dans une zone de chevauchement entre ladite section d'entrejambe (C) et la section arrière de dos (R).

3. Article absorbant selon l'une quelconque des revendications précédentes, dans lequel les moyens d'élastification de jambe (8) se terminent dans la direction longitudinale (9) dans la zone (20, 21) de la section avant d'abdomen (F) et la section arrière de dos (R) dans laquelle des seconds moyens d'élastification (22, 23) sont prévus.

4. Article absorbant (A) selon l'une quelconque des revendications précédentes, dans lequel les premiers moyens d'élastification (6) s'étendent, en étant séparés l'un de l'autre et parallèlement l'un à l'autre et au bord transversal (17, 18), afin d'élastifier la section avant d'abdomen (F) et la section arrière de dos (R).

5. Article absorbant (A) selon l'une quelconque des revendications précédentes, dans lequel la section avant d'abdomen (F) et/ou la section arrière de dos (R) comprend en outre un matériau élastifié (28) prévu sur 1% à 65% de la surface de ladite section avant d'abdomen (F) et/ou de la section arrière de dos (R).

6. Article absorbant selon l'une quelconque des revendications précédentes, dans lequel le degré de répartition des seconds moyens d'élastification (23) dans la section arrière de dos (R) est supérieur au degré de répartition des seconds moyens d'élastification (22) dans la section avant d'abdomen (R).

7. Article absorbant selon l'une quelconque des revendications précédentes, dans lequel la séparation entre les seconds moyens d'élastification (22) de la section avant d'abdomen (F) est de 3 à 20 mm, de préférence de 4 à 15 mm, de préférence de 5 à 10 mm, de préférence de 6 à 8 mm.

8. Article absorbant selon l'une quelconque des revendications précédentes, dans lequel la section d'entrejambe (C) comprend un matériau de fond imperméable au liquide (2) et un matériau de dessus (3) entre lesquels le corps absorbant (4) est disposé, et le matériau de fond (2) et/ou le matériau de dessus (3) forment une saillie (30) qui s'étend au-delà du corps absorbant (4) dans la direction transversale (12) et des deux côtés dudit corps absorbant (4).

9. Article absorbant selon l'une quelconque des revendications précédentes, dans lequel le corps absorbant (4) est au moins partiellement recouvert par un matériau à très forte absorption (5).

10. Article absorbant selon l'une quelconque des revendications précédentes, dans lequel la proportion de surface de la section d'entrejambe (C) par rapport à la surface entière de l'article absorbant (A) est comprise entre 25 et 55%.

11. Article absorbant selon l'une quelconque des revendications précédentes, dans lequel la section d'entrejambe (C) chevauche au moins 12% de la surface de la section avant d'abdomen (F) et au moins 20% de la surface de la section arrière de dos (R) dans un état étendu de l'article absorbant.

12. Article absorbant selon l'une quelconque des revendications précédentes, dans lequel le corps absorbant (4) chevauche au moins 5% de la surface d la section avant d'abdomen (F) et/ou au moins 10% de la surface de la section arrière de dos (R).

13. Article absorbant selon l'une quelconque des revendications précédentes, dans lequel la section avant d'abdomen (F) et/ou la section arrière de dos (R) comprennent en outre un matériau élastifié (28) prévu sur au moins 1% à 65% de la surface de ladite section avant d'abdomen (F) et/ou de la section arrière de dos (R).

14. Article absorbant selon l'une quelconque des revendications précédentes, dans lequel la proportion de surface de la section d'entrejambe (C) par rapport à la surface entière de l'article absorbant (A) est comprise entre 25 et 55%, ladite section d'entrejambe chevauche au moins 12% de la surface de la section avant d'abdomen (F), et au moins 20% de la surface de la section arrière de dos (R) dans un état étendu de l'article absorbant, et dans lequel le corps absorbant (4) chevauche au moins 5% de la surface de la section avant d'abdomen (F) et/ou au oins 10% de la surface de la section arrière de dos (R).

*Fig. 1*

*Fig. 2*

*Fig. 3*

*Fig. 4*

Fig. 5

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009187157 A

- EP 1035818 A